# EUROPÄISCHE PATENTSCHRIFT

(11) **EP 1 250 884 B1**
(45) Veröffentlichungstag und Bekanntmachung des Hinweises auf die Patenterteilung: **28.12.2005**
(21) Anmeldenummer: 01810832.4
(22) Anmeldetag: 28.08.2001
(51) Int. Cl.: A61B 3/16

(54) **Vorrichtung zur Messung des Augeninnendrucks, insbesondere Tonometer**
Apparatus for measuring the intraocular pressure, in particular a tonometer
Dispositif pour mesurer la pression intraoculaire, particulièrement un tonomètre

(30) Priorität: 19.04.2001 CH 7102001
(43) Veröffentlichungstag der Anmeldung: 23.10.2002
(73) Patentinhaber: SMT Swiss Microtechnology AG, 2562 Port (CH)
(72) Erfinder: Kanngiesser, Hartmut, 8038 Zürich (CH)
(74) Vertreter: Roshardt, Werner Alfred

(56) Entgegenhaltungen:
- WO-A-00/71982
- WO-A-01/05299
- US-A- 4 089 329
- US-A- 4 922 913
- US-A- 4 922 914
- US-A- 5 032 020
- US-A- 5 217 015

## Beschreibung

### Technisches Gebiet

Die Erfindung betrifft eine Vorrichtung zur Messung des Augeninnendrucks, insbesondere ein Tonometer gemäss dem Oberbegriff des Patentanspruchs 1.

### Stand der Technik

Zur Messung des Augeninnendrucks sind eine Reihe von Geräten z. B. nach Goldmann, Machay und Marg bzw. nach Robert (US-A 5 032 020; EP-A 0 327 693; CH-A 673 760; WO-A 00/71982; US-A 4,922,914, US-A 4,922 914; WO 01/05299) bekannt. Bei den bekannten Tonometern wurde der Augeninnendruck durch Plandrücken eines kleinen vorgegebenen Bereichs auf dem Auge ermittelt. Die bekannten Messverfahren waren alle mit systematischen Messfehlem behaftet.

### Darstellung der Erfindung

### Aufgabe der Erfindung

Aufgabe der Erfindung ist es, eine Vorrichtung zur Bestimmung des Augeninnendrucks zu schaffen, welche den tatsächlich im Auge herrschenden Innendruck möglichst genau ermittelt.

### Lösung der Aufgabe

Die Aufgabe wird dadurch gelöst, dass im Gegensatz zu den bekannten Tonometern die auf die Augenoberfläche aufzulegende Anlegefläche eines Basiskörpers der Kontur einer Standardoberfläche eines Standardauges mit vorgegebenem Innendruck bis auf eine Toleranz anschmiegbar angepasst ist und in der Anlegefläche eine drucksensitive Einheit wirksam ist.

Bei der Erfindung wird nun im Gegensatz zu den bekannten Tonometem nicht mehr auf der Hornhaut eine plane Messfläche (Applanationsbereich) erzeugt. Bei den bekannten Tonometern hatte man nämlich fälschlicherweise angenommen, dass die Hornhaut durch planes Verformen entspannt wird.

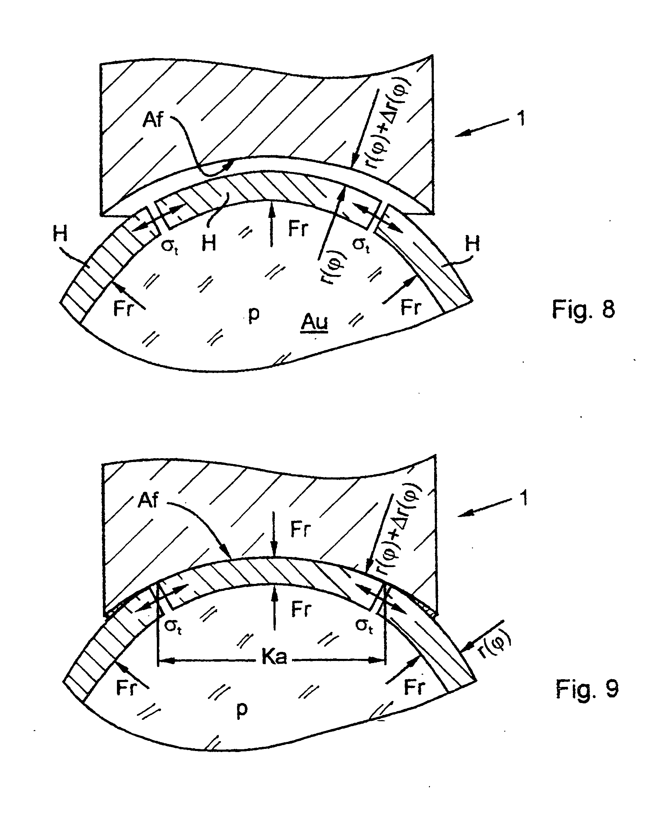

Erfindungsgemäss wird nun davon ausgegangen, dass die Hornhaut des menschlichen Auges einen natürlichen Krümmungsradius hat, der sich als Funktiondes Augeninnendrucks nur unwesentlich ändert und auch bei einem Augeninnendruck Null, also bei gleichem Druck beidseits der Hornhaut, erhalten bleibt. Zur Erläuterung des erfindungsgemässen Grundgedankens wird von einem Auge **Au** ausgegangen, wie es in **Figur 8** dargestellt ist, welches unberührt und frei von externen Kräften ist. Im Auge **Au** herrscht ein zu messender Augeninnendruck **P.** Dieser Innendruck erzeugt Kräfte **F**_{**r**}**,** die radial auf die Augenhornhaut **H** wirken und in dieser tangentiale Spannung σ_{**t**} verursachen.

In einem nächsten Gedankenschritt wird davon ausgegangen, dass durch Anlegen der Anlegefläche **Af** die durch den Augendruck erzeugten und radial nach aussen gerichteten Kräfte **F**_{**r**} direkt in die Anlegefläche **Af** weitergeleitet und somit aus der Hornhaut eliminiert werden, wie in **Figur 9** dargestellt ist. Der Bereich, auf dem die Anlegefläche **Af** aufliegt, wird Konturanpassungsbereich **Ka** genannt. Im Bereich **Ka** passt sich die Kontur der Hornhaut **H** derjenigen der Anlegefläche **Af** an. Die Kontur der Anlegefläche **Af** definiert also die Verformung der Hornhaut **H.** Ausserhalb dieses Bereichs **Ka** wirken auf die Hornhaut **H** weiterhin die radialen Kräfte **F**_{**r**} des Augeninnendrucks. Die durch die radialen Kräfte **F**_{**r**} entstehenden tangentialen Spannungen **σ**_{**t**} pflanzen sich in den Bereich **Ka** hinein fort. Im Idealfall herrschen dann in diesem Bereich **Ka** nur noch die tangentialen Spannungen **σ**_{**t**} und keine radialen Kräfte **F**_{**r**} mehr. Die den Augeninnendruck repräsentierenden radialen Kräfte **F**_{**r**} werden somit ohne Änderung in den Bereich **Ka** auf die Anlegefläche **Af** übertragen. Die tangentialen Spannungen **σ**_{**t**} verändern bei fehlenden radialen Kräften **F**_{**r**} die natürliche Krümmung der Hornhaut **H** in Richtung grösserer Radien. Für eine exakte Augeninnendruckmessung ist demzufolge keine Applanation, sondern ein Formschluss mit oben definierter Verformung der Hornhaut herzustellen (was mit bekannten Tonometem nicht machbar war).

Der Aufbau der Hornhaut **H** ist mehrschichtig und besteht aus dem Epithel, der Bowmanschen Membran, dem Stroma, der Descemetschen Membran und dem Endothel. Eine Optimierung der oben beschriebenen Verformung kann entweder empirisch durch Messreihen (wie weiter unten beschrieben ist) oder durch Modellrechnungen erfolgen, die jedoch wegen des kaum modellierbaren, komplexen Aufbaus der Hornhaut **H** mit grossen Unsicherheiten behaftet sind.

Erfindungsgemäss wird nun mit einer Anlegefläche gearbeitet, welche der natürlichen Krümmung der Augenhornhaut im definiert verformten Zustand entspricht. Unter definiert verformt wird der Zustand der Hornhaut **H** verstanden, in den sie gelangt, wenn die radialen Kraftkomponenten **F**_{**r**} eliminiert werden; die tangentialen Spannungen **σ**_{**t**} hingegen weitestgehend erhalten bleiben, wie bereits weiter oben ausgeführt wurde. Erreicht wird dies durch Anlegen der Anlegefläche **Af** auf die Hornhaut **H** deren Oberflächenkontur der Standardoberfläche eines Standardauges bis auf eine Toleranz angepasst ist. Standardoberfläche und Standardauge entsprechen den Eigenschaften und Abmessungen des durchschnittlichen menschlichen Auges. Messungen haben nämlich ergeben, dass sich die Abmessungen menschlicher Augen nur unwesentlich von einander unterscheiden. Geringfügig grössere Unterschiede ergeben sich, nachdem das Auge einer refraktiven Chirurgie der Hornhaut (z.B. mit Skalpel oder Laser) unterzogen worden ist.

Die Anlegefläche wird vorzugsweise als Oberfläche eines Basiskörpers ausgebildet. In der Regel ist im Zentrum der Anlegefläche eine drucksensitive Einheit angeordnet, deren Anlegefläche in die Kontur der Anlegefläche des Basiskörpers mit einer Übergangstoleranz übergeht. Die Übergangstoleranz sollte derart gewählt werden, dass keine Sprungstellen vorhanden sind und der Oberflächenverlauf der Anlegefläche des Basiskörpers und der drucksensitiven Einheit durch ein- und dieselbe mathematische Gleichung beschreibbar sind. Die einfachsten in einander übergehenden Konturen wären Konturen mit ein- und demselben Krümmungsradius, ausgehend von und ein- und demselben Zentrum (sphärische Kontur); es sind aber auch asphärische Konturen möglich.

Eine derartige Übergangstoleranz ist der Idealfall, der sich praktisch nur mit grossem Aufwand einhalten lässt. Gerade noch tolerierbar ist ein Tiefenversatz von einem Millimeter und ein Winkelversatz der Kurventangenten am Rand des Übergangs von 10°. Anzustreben ist jedoch ein Versatz von 0 Mikrometer und ein Winkelversatz von 0°.

Der Unterschied der Krümmungsradien von der Anlegefläche zur Standardoberfläche, der sich durch die definierte Verformung ergibt, ist so gering, dass sich ein genügend grosser Konturanpassungsbereich im Allgemeinen schon bei einer Aufsetzkraft Null durch die Wirkung des Kapillardrucks der Tränenflüssigkeit ergibt. Der Konturanpassungsbereich kann allenfalls auch durch eine zusätzlich von aussen angelegte Kraft (Aufsetzkraft) vergrössert werden. Wie die ideale Grösse dieses Bereiches zu bestimmen ist, wird weiter unten erklärt. Für den Unterschied der Krümmungsradien kann ein gewisser Bereich zugelassen werden.

Der Unterschied ist derart zu wählen, dass sich ein möglichst schmaler, jedoch hinreichend grosser Spalt zwischen Anlegefläche und Hornhautoberfläche ergibt. Je schmaler der Spalt desto grösser ist der wirkende Kapillardruck und desto besser ist eine "Kopplung" zwischen Hornhaut und Anlegefläche. Je grösser jedoch der Spalt ist desto mehr Tränenflüssigkeit kann der Spalt aufnehmen. Zuviel Tränenflüssigkeit müsste nämlich abgetupft werden, was einen erhöhten Aufwand bedeutet.

Steht der Tränenfilm am Rand der Anlegefläche über, so ist der Krümmungsradius des Tränenfilm-Luftübergangs nach aussen gewölbt und der Kapillardruck positiv; d. h., er wirkt abstossend zwischen Anlegefläche und Hornhaut. Um dann einen genügend grossen Bereich mit Konturanpassung zu schaffen, muss eine grosse zusätzliche Anlegekraft aufgewendet werden, welche in unzulässiger Weise den Augeninnendruck erhöhen würde. (Diese Auswirkung wäre analog zu einem zu grossen Kurvenradius der Anlegefläche.) Ist der "Kurvenradius" zu gross, muss, um einen genügend grossen Bereich mit Konturanpassung zu schaffen, ebenfalls eine zusätzliche Anlegekraft aufgewendet werden.

Für die Messung am menschlichen Auge sind Anlegeflächen mit Radien zwischen 8 mm und 11 mm geeignet. Experimentell gute Ergebnisse für die Messung auf gesunden und chirurgisch nicht veränderten Hornhäuten mit unterschiedlichen Homhautradien wurden bei Anlegeflächen mit Radien zwischen 9,5 mm und 10,5 mm erreicht [siehe Figur 10]. Fallweise (bei pathologisch und/oder chirurgisch veränderten Hornhäuten) können auch andere Radien mit Vorteil zum Einsatz kommen.

Die Anlegefläche ist konkav (nach innen gewölbt) ausgebildet. Der Oberflächenverlauf kann sphärisch, aber auch asphärisch sein, wie bereits oben erwähnt.

Die drucksensitive Einheit kann nun eine Membran haben, deren Kontur in die Kontur der Anlegefläche möglichst sprunglos übergeht. Die Membranrückseite ist mit einem druckübertragenden Medium beaufschlagt, welches den Druck zu einem im Randbereich des Basiskörpers angeordneten Drucksensor überträgt. Als druckübertragendes Medium kann ein inkompressibles Medium (Material) dienen. Ein derartiges Medium ist z. B. eine Flüssigkeit, ein Gel oder eine flexible Vergussmasse. Vorzugsweise wird man diese Medien im optischen Bereich transparent wählen, damit sie eine Betrachtung durch die Vorrichtung hindurch auf oder ins Auge nicht stören. Da auch der Drucksensor ausserhalb des Beobachtungs- oder Behandlungsstrahlenganges liegt, erfolgt keine optische Beeinträchtigung.

Auf eine Anordnung mit einem Druckübertragungsmittel kann zum einen verzichtet werden, wenn keine Betrachtung gewünscht wird oder zum anderen, wenn ein derart kleiner Drucksensor verwendet wird, der den optischen Beobachtungsstrahlengang nicht merklich stört. Ein derartiger Sensor kann ein Halbleitersensor sein. Den Sensor kann man auch noch elektrisch mit einem Verarbeitungschip koppeln. Es ist dann z. B. eine passive telemetrische Übertragung der ermittelten Augeninnendruckwerte (-verlauf), vorzugsweise über einen vorgegebenen Zeitraum möglich.

Die gesamte Messvorrichtung kann auch so klein ausgeführt werden, dass sie quasi als "Kontaktlinse" auf dem Auge zu tragen ist. Diese "Kontaktlinse" wird dann über die Tränenflüssigkeit gehalten und "konturangepasst" an die Augenoberfläche "herangezogen". Die der Anlegefläche abgewandte Linsenfläche kann dann entsprechend der gewünschten Sehschärfe für den Patienten oder für ein Abbildungssystem eines Spaltlampenmikroskops oder einer anderen Betrachtungseinheit ausgebildet werden.

Der Basiskörper kann aber auch durchsichtig und abbildungsneutral ausgebildet werden.

Die Anlegefläche mit wirksamer drucksensitiver Einheit muss zur Augendruckmessung auf die Hornhaut aufgelegt werden. In der Regel ist die Hornhaut mit einem Tränenfilm überzogen. Teile dieses Tränenfilms können an der Anlegefläche haften bleiben und würden bei einer nachfolgenden Messung auf ein anderes Auge übertragen, was zu einer Infektion führen könnte. Es wird deshalb vorgeschlagen, einen sterilen aufsetzbaren Aufsatz zu verwenden, der nach der Messung entweder weggeworfen wird oder sterilisiert werden kann. Der Aufsatz ist derart ausgebildet, dass über der drucksensitiven Einheit zu liegen kommendes Material derart dünn ausgebildet ist, dass zwar eine Stabilität gegen Zerreissen gegeben ist, jedoch beim Anliegen der Druck von der Augenhornhaut ohne Verlust an die drucksensitive Einheit **(9)** übertragbar ist.

Wie bereits oben ausgeführt, erfolgt der Kraftschluss der Anlegefläche mindestens zum Teil durch Kapillarkräfte. Beim Kapillardruck spielt die Menge vorhandener Tränenflüssigkeit eine ausschlaggebende Rolle. Es ist oben ausgeführt, dass zu viel Tränenflüssigkeit abgetupft werden sollte. Dieses aufwendige Abtupfen kann durch die Verwendung einer "Tupfereinheit" eliminiert werden. Diese Tupfereinheit ordnet man dann am Rand der Anlegefläche an. Auf die Ausgestaltung dieser Einheit wird unten genauer eingegangen.

Um gute Messresultate zu erhalten sollte sich die Anlegefläche während der Messung nicht gegenüber dem Auge bewegen. Da das Patientenauge nicht betäubt ist, kann eine Augenbewegung jederzeit, auch vom Patienten ungewollt erfolgen. Es wird deshalb eine eine vorgegebene Steifigkeit aufweisende Einrichtung vorgesehen, welche die die Anlegefläche aufweisende Einheit mit einem Fixierungsort verbindet. Die Steifigkeit ist derart zu wählen, dass die Einheit bei auf das Auge aufgelegter Anlegefläche dann wenigstens kleine Augenbewegungen mitmacht.

### Kurze Beschreibung der Zeichnungen

Im Folgenden werden Beispiele der erfindungsgemässen Vorrichtung zur Messung des Augeninnendrucks anhand der nachfolgenden Zeichnungen näher erläutert. Weitere Vorteile der Erfindung ergeben sich aus dem Beschreibungstext. Es zeigen:
- Fig. 1: einen Querschnitt durch die erfindungsgemässe Vorrichtung,
- Fig. 2: einen Querschnitt durch eine Variante zu der in **Figur 1** dargestellten Vorrichtung,
- Fig. 3: einen Querschnitt durch eine weitere Variante zu den in den **Figuren 1** und **2** dargestellten Vorrichtungen,
- Fig. 4: eine Ausführungsvariante mit wegnehmbarem sterilen Aufsatz,
- Fig. 5: eine Ausführungsvariante zu der in **Figur 4** gezeigten Ausführungsform,
- Fig. 6: eine Ausführungsvariante mit einer elastischen Halterung,
- Fig. 7: eine Ausführungsvariante mit einer Tupfereinheit zur Aufnahme von überschüssiger Tränenflüssigkeit,
- Fig. 8: eine schematische Darstellung zur Erklärung der in und auf die Hornhaut wirkenden Kräfte bzw. Spannungen infolge des Augeninnendrucks,
- Fig. 9: eine analoge Darstellung zu **Figur 8** mit aufgelegter erfindungsgemässer Vorrichtung und
- Fig. 10: ein Messdiagramm zur Ermittlung der optimalen Kontur der Anlegefläche der erfindunsgemässen Vorrichtung.

### Wege zur Ausführung der Erfindung

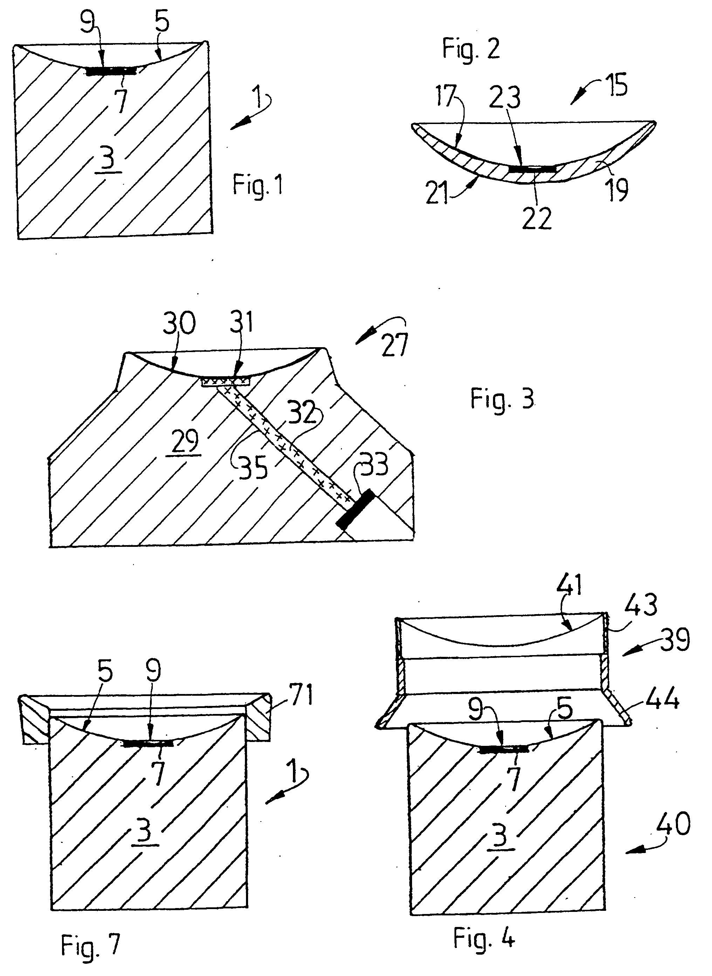

Die in **Figur 1** dargestellte Vorrichtung **1** zur Messung des Augeninnendrucks hat einen Basiskörper **3,** der eine auf die Augenoberfläche aufzulegende Anlegefläche **5** hat. Die Kontur der Anlegefläche **5** ist der Standardoberfläche eines Standardauges mit vorgegebenem Innendruck bis auf eine Toleranz angepasst. Erklärend ist hier zu erwähnen, dass sich der Krümmungsradius des natürlichen Auges in Abhängigkeit des Augeninnendruckes nur sehr wenig ändert. Eine Standardoberfläche des unbewehrten Auges wird typischerweise in Folge des Innendruckes mit einem Krümmungsradius von 7,5 mm angenommen. Der Krümmungsradius der konkaven (nach innen gewölbten) Anlegefläche **5** liegt typischerweise zwischen 8 mm und 11 mm, vorzugsweise zwischen 9, 5 mm und 10, 5 mm. Wie bereits oben ausgeführt, kann die Anlegefläche **5** sphärisch oder auch asphärisch ausgebildet sein.

In der Anlegefläche **5,** hier beispielsweise zentrisch, also im tiefsten Ort, ist eine drucksensitive Einheit, ein Drucksensor **7,** ausgebildet. Als Drucksensor **7** wird man vorzugsweise aufgrund z. B. der kleinen Abmessungen und des minimalen Gewichts einen Halbleitersensor verwendet. Der Drucksensor kann nun über (nicht dargestellte) Signalleitungen mit einer (nicht dargestellten) externen Verarbeitungsschaltung verbunden werden. Vorzugsweise wird jedoch ein Verarbeitungschip gleich in den Drucksensor integriert. Der Verarbeitungschip sollte dann beispielsweise derart ausgestaltet sein, dass eine passive telemetrische Übertragung zu einem entfernten (nicht dargestellten) Auswertegerät möglich ist. Die Oberfläche **9** des Drucksensors **7** bzw. der integrierten Einheit (Drucksensor + Verarbeitungschip) geht ohne Sprung in die Oberflächenkontur der Anlegefläche **5** über.

Damit die Hornhaut **H** im Zentrum der Anlegefläche **5,** wo der Drucksensor **7** sitzt, nur tangentiale Spannungen **σ**_{**t**} aufweist und keine radialen Kräfte **F**_{**r**} mehr vorhanden sind, benötigt man einen Kurvenradius der Anlegefläche **5** im auf das Auge aufgelegten Zustand von **r(ϕ) + A r(ϕ)**, wobei r der Abstand vom Augenmittelpunkt eines Oberflächenpunktes auf der Anlegefläche **Af** und **ϕ** der von der optischen Augenachse abweichende Winkel des Punktes ist. Vorausgesetzt wird hier ein sphärisches Augenmodell mit Symmetrie zur optischen Achse, was in der Realität nicht erfüllt ist; für diese Erklärungen aber vernachlässigt werden kann. Bei einer Konturanpassung wird im Auge ein bestimmtes Volumen verdrängt, das zu einer Druckerhöhung im Auge und als Folge dann zu einem Messfehler führen würde. Dieses Volumen sollte so klein wie möglich sein. Es sollte höchstens so gross wie bei einer Standard Goldmann-Untersuchung und somit kleiner als 0,58 mm³ sein. Für die oben angegebenen Radien der Anlegefläche **5** zwischen 9,5 mm und 10,5 mm ist ein Durchmesser des Konturanpassungsbereichs von 4 mm ausreichend. Das verdrängte Volumen bei einer Augendruckmessung liegt dann zwischen 0,38 mm³ und 0,51 mm³. Bei grösseren Radien wird das verdrängte Volumen grösser und die dadurch verursachte Druckerhöhung führt zu Messfehlern. In Fig 10 ist bei einem Radius von 12 mm bereits erkennbar, dass die Eichkurve nie flach verläuft. D. h., der Druck ist über den gesamten Bereich abhängig von der Grösse des Bereichs mit Konturanpassung **Ka**. Bei planer Anlegefläche (Fig. 10) ist das Verhalten noch ausgeprägter.

Sind die oben geführten Bedingungen für einen von radialen Kraftkomponenten **F**_{**r**} befreiten Bereich des Drucksensors **7** gegeben, so wird ein realer Augeninnendruck gemessen. D. h. alle auf den Drucksensor **7** wirkenden Kräfte wirken senkrecht auf dessen Oberfläche. Der Drucksensor **7** kann wie herkömmliche Drucksensoren mit einem geeichten Druck eines Gases oder einer Flüssigkeit zu Eichzwecken beaufschlagt werden.

Zur Ermittlung des für eine Messung benötigten idealen Krümmungsradius und einer dazugehörigen optimalen Grösse der Anlegefläche **5** wird, wie nachfolgend angeführt, vorgegangen. Hierzu nimmt man eine Eichkurve, wie in **Figur 10,** dargestellt auf. In dieser Eichkurve wird der gemessene Druck als Funktion der Grösse des Bereichs **Ka** mit Konturanpassung bei verschiedenen Konturen der Anlegefläche mit dem Radius **R** der Anlegefläche als Parameter dargestellt. Für ein Fehler minimiertes Messen muss ein Radius ausgewählt werden, bei dem die Kurve in **Figur 10** in einem möglichst grossen Bereich flach verläuft. D.h. bei dem die Vorrichtung denselben Druck misst. Die Toleranzwerte für die Flächenbereiche sind mit **T** gekennzeichnet, wobei der Index den jeweiligen dazu gehörenden Radius der Anlegefläche angibt. Das Fehlen von T₁₂ und Tₚₗₐₙ zeigt an, dass die Kurven in keinem Bereich plan verlaufen und somit zur Innendruckmessung am menschlichen Auge nicht verwendbar sind.

Bei der Vorrichtung **1** handelt es sich um eine preisgünstige Ausführung, welche vorzugsweise nur zur Druckmessung dient. Auf eine gute optische Transmission z. B. für Beobachtungszwecke im Auge wird man hier keinen Wert legen; kann aber selbstverständlich vorgenommen werden. Der Basiskörper **3** kann somit aus einem nicht transparenten Material bestehen. Die Vorrichtung **1** wird in der Regel zusammen mit einer sogenannten Spaltlampe eingesetzt und von der behandelnden Person entweder in der Hand gehalten und gegen das Auge des Patienten gedrückt oder die Vorrichtung ist auf einer Spaltlampe montiert und wird durch deren Justiereinrichtung gezielt auf das Auge gebracht Die Vorrichtung kann jedoch eine eigene Justiereinrichtung und Kraftbeaufschlagung (Aufsetzkraft) beinhalten und verbunden mit der Spaltlampe auf das Auge aufgebracht werden.

Die Vorrichtung kann auch eine eigene Justiereinrichtung, eine Kraftbeaufschlagung sowie eine Beobachtungseinheit beinhalten und dann unabhängig von einer Spaltlampe auf das Auge aufgebracht werden.

**Figur 2** zeigt eine Variante zur Vorrichtung **1.** Die in **Figur 2** gezeigte Vorrichtung **15** ist in der Art einer Kontaktlinse ausgebildet. Auch sie hat eine Anlegefläche **17,** deren Kontur der Standardoberfläche eines Standardauges bis auf eine Toleranz, wie oben beschrieben, angepasst ist. Der Basiskörper **19** der Vorrichtung **15** ist aus transparentem Material gefertigt und entspricht weitgehend demjenigen von "weichen" bzw. "harten" Kontaktlinsen. Die der Anlegefläche **17** abgewandte Fläche **21** ist nach optischen Kriterien betreffend der zu erreichenden Korrektur der Sehschärfe für den betreffenden Patienten gestaltet. Ist keine Korrektur erforderlich, so kommt eine optisch neutrale Kontaktlinse zum Einsatz. Die Kraft zum Erreichen des Formschlusses zwischen "Kontaktlinse" und Hornhaut wird durch den Kapillardruck des Tränenfilms aufgebracht. Auch ist die Anordnung des Drucksensors **22** analog zur Anordnung in der Vorrichtung **1** gewählt; d. h. die Oberfläche **23** des Drucksensors **22** geht (möglichst) ohne Sprung in die Anlegefläche **17** über.

Eine weitere Variante einer Messvorrichtung **27** zu den in den **Figuren 1** und **2** gezeigten Vorrichtungen **1** und **15** ist in **Figur 3** dargestellt. Die Vorrichtung **27** hat einen vorzugsweise transparenten Basiskörper **29.** Die Anlegefläche **30** ist analog zu den Anlegeflächen **5** und **17** ausgebildet Eine drucksensitive Einheit hat hier eine Mernbran **31** in der Anlegefläche **30,** ein druckübertragendes Medium **32** und einen im Randbereich des Basiskörpers **29** angeordneten Drucksensor **33.** Die Wölbung der Membran **31** geht ohne Sprung in die Kontur der Anlegefläche **30** über. Das Druckübertragungsmedium **32** ist inkompressibel, z. B. eine Flüssigkeit, ein Gel oder eine flexible Vergussmasse. Das Medium **32** ist abgeschlossen in einem Hohlraumsystern **35**. Etwaige Volumenausdehnungen können wie beispielsweise in der WO 00/71982 beschrieben, kompensiert werden. Das Medium **32** sowie auch den Basiskörper **29** wird man vorzugsweise für optische Strahlung transparent ausbilden, um eine Betrachtung des Auges durch die Vorrichtung **27** hindurch vornehmen zu können. Auch wird man den Brechungsindex des Mediums **32** und des Materials des Basiskörpers **29** annähernd gleich wählen. Die Breite des Basiskörpers **29** ist grösser gewählt als diejenige des Basiskörpers **3,** um einen grösseren Beobachtungwinkel zu erhalten.

Eine Vorrichtung **40** zur Messung des Augendrucks analog der in **Figur 1** dargestellten Vorrichtung ist in **Figur 4** gezeigt. Entgegen der Ausführung in **Figur 1** ist hier ein abnehmbarer Aufsatz **39** vorhanden. Dieser Aufsatz ist steril und steril verpackt und wird zur Augenuntersuchung auf einen Grundkörper **3**, der mit der Vorrichtung **1** in Figur **1** identisch ist, aufgesetzt. In einer besonderen Ausführungsform ist dieser Aufsatz sterilisierbar ausgebildet, damit er jeweils nach dem Sterisilieren für weitere Untersuchungen verwendbar ist. In der Regel wird man aber den Aufsatz als preisgünstiges Wegwerfelement ausbilden. Der teure Drucksensor **7** verbleibt immer im Grundkörper **3** und ist bei der Augeninnendruckmessung durch den Aufsatz **39** abgedeckt.

Der Aufsatz **39** ist derart ausgebildet, dass er auf den Grundkörper **3** (Vorrichtung **1**) abnehmbar aufsteckbar ist. Ausgehend von einem kreiszylindrischen Querschnitt des Grundkörpers **3** ist auch der Aufsatz **39** in seinen Innenkonturen und damit auch Aussenkonturen kreiszylindrisch ausgebildet. Der Aufsatz **39** hat einen Rahmen, dessen Innendurchmesser um eine klemmende Spieltoleranz grösser ist als der Aussendurchmesser des Basiskörpers **3** des Grundkörpers **3**. Nach oben ist der Aufsatz **39** durch eine dünne Membran **41** abgeschlossen. Die Membran **41** kann aus Kunststoff oder Metall bestehen. Ihre Oberflächenkontur entspricht derjenigen der Auflegefläche **5.** Der Übergang vom Rahmen **42** zur Membran **41** erfolgt über eine Randverstärkung **43.** Die Verstärkung **43** erhöht die Stabilität des Aufsatzes **39** und erleichtert die Montage der Membran **41.** An den kreiszylindrischen Rahmen **42** nach unten in **Figur 4** anschliessend ist ein nach aussen stehender ringförmiger Kragen angeordnet. Der Kragen **44** soll einerseits die Handhabung erleichtern und zudem verhindern, dass eventuell tropfende Tränenflüssigkeit auf den Grundkörper **3** gelangen kann.

Zum Aufsetzen des Aufsatzes **39** auf den Grundkörper **3** werden Aufsatz **39** und Grundkörper **3** in der in **Figur 4** gezeigten Lage gehalten. Auf die Anlegefläche **5** werden einige Tropfen einer sterilen Flüssigkeit gegeben und anschliessend der Aufsatz **39** aufgedrückt. Ein kleiner Zwischenraum zwischen Anlegefläche **5** und der Membranrückseite ist vollständig mit der Flüssigkeit gefüllt. Überschüssige Flüssigkeit wird bei der Montage zur Verstärkung **43** hin verdrängt. Durch den dünnen Flüssigkeitsfilm hindurch ist eine einwandfreie Kraftübertragung auf den Drucksensor **9** gegeben.

Anstelle einer sterilen Flüssigkeit kann auch ein steriles Gel verwendet werden. Auf Flüssigkeit und Gel kann auch verzichtet werden, wenn die sich berührenden Oberflächen entsprechende Eigenschaften aufweisen [ausreichende Adhäsion (elek trostatisch, kapillare Wirkung, ...), ...].

Der Basiskörper **3** und der auf ihn aufsetzbare Aufsatz **39** müssen nicht kreiszylindrisch ausgebildet sein; andere Querschnitte sind möglich.

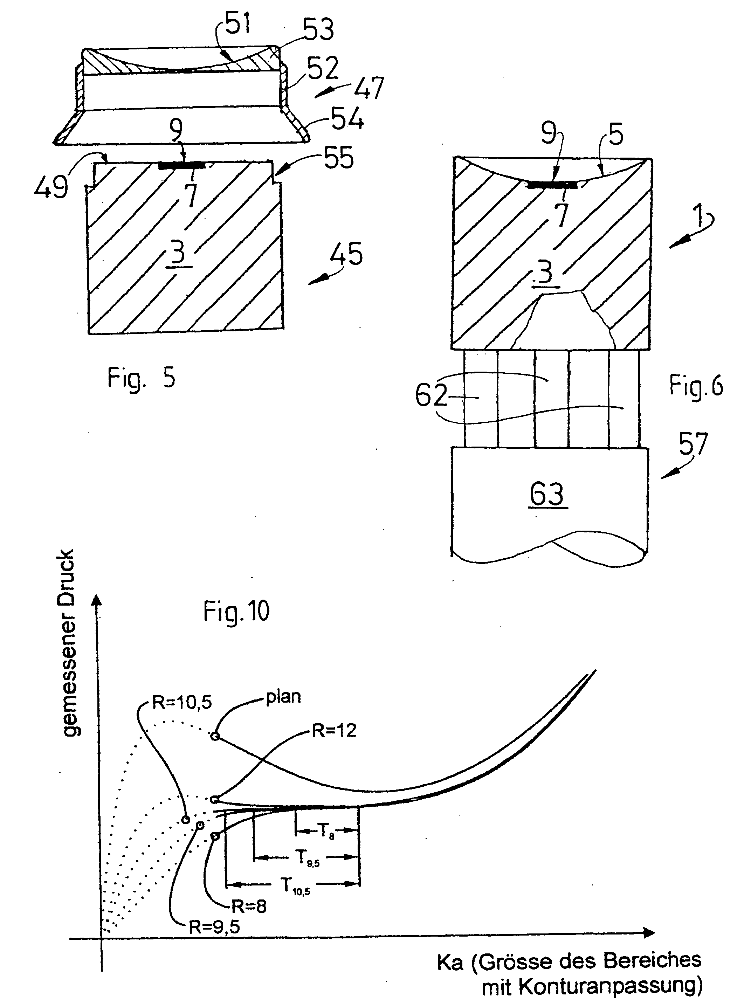

Eine weitere Ausführungsvariante **45** zeigt **Figur 5**. Auch hier ist analog zur Ausführungsvariante in **Figur 4** ein Grundkörper **3** und ein aufsetzbarer, steriler Aufsatz **47** vorhanden. Auch hier hat der Aufsatz **47** einen kreiszylindrischen Rahmen **52** und einen Kragen **54** der analog zum Kragen **44** ausgebildet ist. Im Gegensatz zur Ausführung in **Figur 4** ist hier jedoch der Drucksensor **7** in eine ebene Fläche **49** eingesetzt. Diese ebene Fläche **49** lässt sich nämlich preisgünstiger herstellen als die gewölbte Fläche **5** des Grundkörpers **3**. Der Aufsatz **47** hat jetzt jedoch im Gegensatz zur Membran **41** des Aufsatzes **39** einen Körper **53** aus flexiblem Material, dem eine Anlegefläche **51** angeformt ist. Der flexible Körper **53** hat in seinem über dem Drucksensor zu liegen kommenden Bereich eine äusserst dünne Wandstärke. Je dünner desto besser. Die minimale Wandstärke wird lediglich durch mechanische Anforderungen gegen Zerreissen bestimmt. Aufgrund der extrem dünnen Wandstärke über dem Drucksensor **7** wird der Druck der von der Anlegefläche **51** vom Auge abgeleitet wird ohne Verlust an den Drucksensor **9** weitergeleitet. Dies ist genau dann der Fall, wenn der Druck, der nötig ist, um flexibles Material des Körpers **53** aus dem Bereich mit Konturanpassung in den Bereich ohne Konturanpassung zu verdrängen, viel grösser ist als der Druck der bei der Konturanpassung entstanden ist.

Auch hier wird analog zur Ausführung in **Figur 4** eine sterile Flüssigkeit oder ein steriles Gel bei der Montage auf die ebene Fläche **49** aufgebracht. Überschüssige Flüssigkeit oder überschüssiges Gel wird bei der Montage in einen Absatz **55** verdrängt. Auch hier kann gemäss obigen Ausführungen auf Flüssigkeit bzw. Gel verzichtet werden.

Die Anlegefläche **51** kann mit einer Beschichtung versehen oder mit einer Membran überzogen werden. Hierdurch kann beispielsweise eine gute Benetzung durch den Tränenfilm erreicht werden. Durch diese Massnahme lassen sich dann die Oberflächeneigenschaften im Hinblick auf eine einfache und reproduzierbare Messung günstig beeinflussen.

Die oben beschriebenen Aufsätze **39** und **47** können auch derart ausgestaltet werden, dass sie auf den Basiskörper **27,** wie er in **Figur 3** dargestellt ist, aufgesetzt werden können.

Die Anlegefläche **5, 30, 41** bzw. **51** soll möglichst nicht auf der Augenoberfläche gleiten, da dies störende Druckspitzen (Spikes) im Messsignal hervorruft. D. h., es soll eine Konstruktion gefunden werden, bei der die Anlegefläche bzw. die die Anlegefläche aufweisende Einheit wenigstens kleine Augenbewegungen (± 1 mm) mitmacht Derartige Anforderungen lassen sich mit einer flexibel ("schwabbelig") aufgehängten, jedoch eine vorgegebene Steifigkeit aufweisende Einheit erreichen. Die verbindende Einrichtung **62** zur Einheit muss jedoch noch so stabil sein, dass sich bei einem freien Halten kein Abknicken nach unten ergibt

Eine derartige Messvorrichtung ist in **Figur 6** skizziert. Die Vorrichtung **1** ist hier über drei flexible Stützen **62** als verbindende Einrichtung mit einem Griffelement **63** als Fixierungsort verbunden. Neben dem Griffelement **63** können auch andere Fixierungsorte, wie Stative, beliebige Halterungen, ... verwendet werden. Die flexiblen Stützen können aus Metallfolie, Gummi, Silikon oder anderen flexiblen Materialien bestehen. Anstelle der Vorrichtung **1** können selbstverständlich auch die in den **Figuren 3, 4** und **5** dargestellten Vorrichtung flexibel gehalten werden.

Produziert ein Auge zu viel Tränenflüssigkeit, so kann dies mit einer in **Figur 7** gezeigten Massnahme verringert werden. Auf die in **Figur 1** gezeigte Vorrichtung **1** wird eine auf den Querschnitt und die Form der Vorrichtungen **1, 27, 40** oder **45** angepasste, hier ringförmige Tupfereinheit **71** montiert. Die Tupfereinheit **71** besteht aus einem hygroskopischen, weichen und flexiblen Material. Dieses Material ist derart beschaffen, dass es innerhalb der ersten Sekunden nach dem Aufsetzen auf das Auge eine bestimmte Menge von etwa 100 µl Tränenflüssigkeit aufnehmen kann. Ferner darf die Tupfereinheit **71** die Hornhaut nicht verletzen und muss sich der Kontur der Hornhaut mit einem geringen Kraftaufwand anpassen. Als Material kann beispielsweise ein sich selbst entfaltender Schwamm oder ein Zellstoffgewebe dienen.

Im Gegensatz zur Ausbildung in **Figur 7** kann die Tupfereinheit auch als Teil der Anlegefläche ausgebildet sein. Wird ein grosses Aufnahmevolumen gewünscht kann die Tupfereinheit auch als in den beiden Lidspalten zu liegen kommend ausgebildet werden.

Die Tupfereinheiten können auch an den in den **Figuren 3** bis **6** analog angeordnet werden.

Der Vorteil der oben beschriebenen erfindungsgemässen Vorrichtungen liegt darin, dass gegenüber den bekannten Tonometern zur Messung keine plane Fläche, sondern eine der Hornhaut angepasste Kontur verwendet wird, wodurch die Hornhaut weitest gehend in einen von radialen Kraftkomponenten befreiten Zustand versetzt wird, was eine von den mechanischen Eigenschaften der Hornhaut unabhängige Messung des Augeninnendrucks mit reduzierten Messfehlem erlaubt. Bekannte Tonometer führten in der Regel ein statisches Messverfahren durch; sich dynamisch ändernde Grössen konnten nicht ermittelt werden.

Mit der erfindungsgemässen Vorrichtung lassen sich bevorzugt auch dynamische Grössen ermitteln. Es ist nämlich neben langfristigen Schwankungen des Augeninnendrucks, bedingt durch die Tageszeit bzw. die Aktivität der betreffenden Person, der Augendruck auch kurzzeitig abhängig vom Blutpuls sowie von emotionalen Einflussgrössen bestimmbar. Der zeitliche Verlauf des Augeninnendrucks erlaubt eine Vielzahl medizinischer Aussagen, welche sich nicht allein auf die Ophthalmologie beschränken.

Mit speziellen Ausführungsvarianten der erfindungsgemässen Vorrichtung kann der Augenhintergrund gut beobachtet werden. Diese vorteilhafte Beobachtung des Augenhintergrundes zusammen mit einer Messung des Augeninnendrucks ist in der EP-A 0 327 693 beschrieben. Gegenüber der dort eingesetzten Vorrichtung ist jedoch die erfindungsgemässe auf bedeutend einfachere Art und Weise mit genaueren Messergebnissen zu verwenden.

## Patentansprüche

1. Vorrichtung zur Messung des Augeninnendrucks als Tonometer mit einem Basiskörper **(3; 19; 29),** der eine auf die Augenoberfläche aufzulegende Anlegefläche **(5; 17; 30; 41; 51)** hat, **dadurch gekennzeichnet, dass** in der Anlegefläche **(5; 17; 30; 41; 51)** eine drucksensitive Einheit **(7; 22; 33)** wirksam ist und die Kontur der Anlegefläche **(5; 17; 30; 41; 51)** einer Standardoberfläche eines Auges **(Au)** mit vorgegebenem Innendruck bis auf eine Toleranz anschmiegbar angepasst ist, wobei eine Druckbeaufschlagung der drucksensitiven Einheit **(7; 22; 33)** durch die auf die Anlegefläche **(5; 17; 30; 41; 51)** aufzulegende Augenoberfläche direkt oder indirekt über ein druckübertragendes Medium **(32; 41; 53)** erfolgt, indem bei der direkten Druckbeaufschlagung die drucksensitive Einheit **(7; 22)** konturangepasst in der Anlegefläche **(5; 17)** angeordnet ist und bei der indirekten Druckbeaufschlagung das druckübertragende Medium **(32; 41; 53)** konturangepasst in der Anlegefläche **(30; 51)** liegt und den Druck zur drucksensitiven Einheit **(33; 7)** überträgt.

2. Vorrichtung nach Anspruch 1, **gekennzeichnet durch** eine in der Anlegefläche **(30)** angeordnete Membran **(31)** als Abschluss des druckübertragenden Mediums **(32)** und einen im Randbereich des Basiskörpers **(29)** angeordneten Drucksensor **(33)** als drucksensitive Einheit, wobei das druckübertragende Medium den Druck von der Membranrückseite zum Drucksensor **(33)** überträgt.

3. Vorrichtung nach Anspruch 2, **dadurch gekennzeichnet, dass**
das druckübertragende Medium **(32)** inkompressibel ist und
der Basiskörper **(29)** einen transparenten Bereich aufweist, durch den in oder auf das Auge geblickt werden kann.

4. Vorrichtung nach Anspruch 3, **dadurch gekennzeichnet, dass**
das druckübertragende Medium eine flexible Vergussmasse ist

5. Vorrichtung nach Anspruch 4, **dadurch gekennzeichnet, dass**
die Vergussmasse transparent ist.

6. Vorrichtung nach einem der Ansprüche 1 bis 3, **dadurch gekennzeichnet, dass**
die drucksensitive Einheit **(7; 22)** eine der Kontur der Anlegefläche **(5; 30)** angepasste Oberfläche **(9; 23)** hat,
welche in die Kontur der Anlegefläche **(5; 30)** mit einer Übergangstoleranz übergeht.

7. Vorrichtung nach einem der Ansprüche 1 bis 6, **gekennzeichnet durch,**
einen die drucksensitive Einheit aufweisenden Basiskörper **(19)** mit der Anlegefläche **(17)** und
eine der Anlegefläche **(17)** gegenüberliegende, gewölbte Fläche **(21)**,
welche eine Linsenwirkung zur Betrachtung und/oder Behandlung der Augenoberfläche bzw. des Augenhintergrundes hervorruft
und/oder der zur Sehkraftveränderung des Patienten dient.

8. Vorrichtung nach Anspruch 7, **dadurch gekennzeichnet, dass**
die Rückseite **(21)** des Basiskörpers **(19)** derart ausgebildet ist und sein Gewicht sowie seine Dimensionen derart gewählt sind,
dass er als Sehschärfe korrigierendes Element auf dem Auge eines Patienten längere Zeit tragbar ist.

9. Vorrichtung nach einem der Ansprüche 1 bis 6, **dadurch gekennzeichnet, dass**
der Basiskörper optisch transparent ausgebildet und
seine in einem Beobachtungsstrahlengang liegenden Flächen derart ausgebildet sind, dass er zusammen mit der Brechkraft des Auges eine optisch neutrale Abbildung erzeugt.

10. Vorrichtung nach einem der Ansprüche 1 bis 9, **gekennzeichnet durch**
einen aufsetzbaren Aufsatz **(39; 47)**, mit einer an die Hornhaut anlegbaren Anlegefläche **(41; 51)** zur auswechselbaren sterilen Abschirmung der nicht sterilen Anlegefläche **(5;49)** von der Hornhaut, wobei
über der drucksensitiven Einheit **(9)** zu liegen kommendes Material **(41; 53)** des Aufsatzes **(39; 47)** derart dünn ausgebildet ist, dass
zwar eine Stabilität gegen Zerreissen gegeben ist,
jedoch beim Anliegen der Druck von der Augenhornhaut ohne Verlust an die drucksensitive Einheit **(9)** übertragbar ist.

11. Vorrichtung nach einem der Ansprüche 1 bis 10, **gekennzeichnet durch**
eine aufsetzbare, vorzugsweise auswechselbare, Tupfereinheit **(71),** welche am Rand der Anlegefläche **(5)** angeordnet ist, und
überschüssige Tränenflüssigkeit aufnimmt und aus dem Bereich der Anlegefläche verdrängt,
so dass keine Beeinflussung der Messung entsteht.

12. Vorrichtung nach einem der Ansprüche 1 bis 11, **gekennzeichnet durch**
eine eine vorgegebene Steifigkeit aufweisende Einrichtung **(62),**
welche die die Anlegefläche **(5)** aufweisende Einheit **(3)** mit einem Fixierungsort **(63)** verbindet, wobei
die Steifigkeit derart zu wählen ist, dass die Einheit **(3)** bei auf das Auge aufgelegter Anlegefläche **(5)** wenigstens kleine Augenbewegungen mitmacht.

## Claims

1. Apparatus for measuring intraocular pressure, in the form of a tonometer incorporating a basic element (3; 19; 29) that has a contact surface (5; 17; 30; 41; 51) designed to be placed against the surface of the eye, **characterised in that** a pressure-sensitive unit (7; 22; 33) is operative in the contact surface (5; 17; 30; 41; 51) and the contour of the contact surface (5; 17; 30; 41; 51) is adapted to be able to hug a standard surface of an eye (Au) at a preset internal pressure up to a certain tolerance, and pressure is exerted on the pressure-sensitive unit (7; 22; 33) by the surface of the eye being positioned against the contact surface (5; 17; 30; 41; 51), either directly or indirectly via a pressure-transmitting medium (32; 41; 53), in the case of direct pressure exertion by virtue of the pressure-sensitive unit (7; 22) being arranged in the contact surface (5; 17) in a contour-adapted manner and in the case of indirect pressure exertion the pressure-transmitting medium (32; 41; 53) being situated in the contact surface (30; 51) in a contour-adapted manner and transmitting the pressure to the pressure-sensitive unit (33; 7).

2. Apparatus according to claim 1, **characterised by** a membrane (31) arranged in the contact surface (30) to act as the end piece for the pressure-transmitting medium (32) and a pressure sensor (33) arranged in the rim of the basic element (29) to act as the pressure-sensitive unit, the pressure-transmitting medium transmitting the pressure from the reverse face of the membrane to the pressure sensor (33).

3. Apparatus according to claim 2, **characterised in that**
the pressure-transmitting medium (32) is incompressible and
the basic element (29) features a transparent area through which the interior or surface of the eye can be viewed.

4. Apparatus according to claim 3, **characterised in that** the pressure-transmitting medium is a flexible casting compound.

5. Apparatus according to claim 4, **characterised in that** the casting compound is transparent.

6. Apparatus according to any one of claims 1 to 3, **characterised in that** the pressure-sensitive unit (7; 22) has a surface (9; 23)
which is adapted to the contour of the contact surface (5; 30),
and which merges into the contour of the contact surface (5; 30) with a transitional tolerance.

7. Apparatus according to any one of claims 1 to 6, **characterised by** a basic element (19) incorporating the pressure-sensitive unit with the contact surface (17) and
a convex surface (21) which is situated opposite the contact surface (17) and induces a lens action for observing and/or treating the surface or fundus of the eye
and/or which is used to alter the visual acuity of the patient.

8. Apparatus according to claim 7, **characterised in that**
the reverse (21 ) of the basic element (19) is designed in such a way and its weight and its dimensions are so chosen
that it can be worn for a long period of time on the eye of a patient as an element for correcting the visual acuity.

9. Apparatus according to any one of claims 1 to 6, **characterised in that** the basic element is designed to be optically transparent and
the surfaces thereof that are situated in an observation path are designed in such a way that together with the refractive power of the eye the element generates an optically neutral image.

10. Apparatus according to any one of claims 1 to 9, **characterised by**
a push-on cap (39; 47) incorporating a contact surface (41; 51) adapted to be placed onto the cornea for shielding the non-sterile contact surface (5; 49) from the cornea in a manner that is interchangeable and sterile;
material (41; 53) of the cap (39; 47) that comes to rest over the pressure-sensitive unit (9) being designed to be so thin that
whilst ensuring stability against laceration,
the pressure of the cornea of the eye can be transmitted to the pressure-sensitive unit (9) without any loss upon placement.

11. Apparatus according to any one of claims 1 to 10, **characterised by**
a push-on, preferably interchangeable, swab unit (71) which is arranged on the rim of the contact surface (5), and
receives excess tear fluid and conveys it from the region of the contact surface,
so as not to affect the measurement.

12. Apparatus according to any one of claims 1 to 11, **characterised by**
a device (62) incorporating a preset rigidity,
which links the unit (3) incorporating the contact surface (5) to a fixing location (63);
the rigidity to be selected so that the unit (3) joins in with at least small eye movements when the contact surface (5) is placed upon the eye.

## Revendications

1. Dispositif pour mesurer la pression intraoculaire, se présentant sous forme de tonomètre avec un corps de base (3 ; 19 ; 29), ayant une surface d'application (5 ; 17 ; 30 ; 41 ; 51) à placer sur la surface de l'oeil, **caractérisé en ce que**, dans la surface d'application (5 ; 17 ; 30 ; 41 ; 51), agit une unité (7 ; 22 ; 33) sensible à la pression, et le contour de la surface d'application (5 ; 17 ; 30 ; 41 ; 51) d'une surface standard d'un oeil (Au), ayant une pression intérieure prédéterminée, étant adapté, en épousant la forme, jusqu'au respect d'une certaine tolérance, une sollicitation en pression de l'unité (7 ; 22 ; 33) sensible à la pression se faisant au moyen de la surface oculaire à appliquer sur la surface d'application (5 ; 17 ; 30 ; 41 ; 51), directement ou indirectement, par l'intermédiaire d'un moyen de transmission de pression (32 ; 41 ; 53), **en ce que**, lors de la sollicitation en pression directe, l'unité (7 ; 22) sensible à la pression est disposée, adaptée au contour, dans la surface d'application (5 ; 17) et, en cas de sollicitation en pression indirecte, le moyen de transmission de pression (32 ; 41 ; 53) est placé, adapté au contour, dans la surface d'application (30 ; 51) et transmet la pression à l'unité sensible à la pression (33 ; 7).

2. Dispositif selon la revendication 1, **caractérisé par** une membrane (31), disposée dans la surface d'application (30), faisant office de barrière au moyen de transmission de pression (32), et un capteur de pression (33), disposé dans la zone de bordure du corps de base (29), faisant office d'unité sensible à la pression, le moyen de transmission de pression transmettant la pression de la face arrière de membrane au capteur de pression (33).

3. Dispositif selon la revendication 2, **caractérisé en ce que** le moyen de transmission de pression (32) est incompressible, et
le corps de base (29) présente une zone transparente, à travers laquelle on peut observer dans ou observer l'oeil.

4. Dispositif selon la revendication 3, **caractérisé en ce que** le moyen de transmission de pression est une masse coulée flexible.

5. Dispositif selon la revendication 4, **caractérisé en ce que** la masse coulée est transparente.

6. Dispositif selon l'une des revendications 1 à 3, **caractérisé en ce que** l'unité sensible à la pression (7 ; 22) présente une surface (9 ; 23), adaptée au contour de la surface d'application (5 ; 30), qui se transforme, avec une tolérance de transition, en le contour de la surface d'application (5 ; 30).

7. Dispositif selon l'une des revendications 1 à 6, **caractérisé par** un corps de base (19), présentant l'unité sensible à la pression et ayant la surface d'application (17), et
une surface bombée (21) opposée à la surface d'application (17),
qui provoque un effet de lentille, pour l'observation et/ou le traitement de la surface de l'oeil ou de l'arrière-plan de l'oeil,
et/ou sert à la modification du pouvoir visuel du patient.

8. Dispositif selon la revendication 7, **caractérisé en ce que** la face arrière (21) du corps de base (19) est réalisée de manière telle, et son poids, ainsi que ses dimensions, sont choisis de manière telle,
qu'il est susceptible d'être porté sur l'oeil d'un patient, en tant qu'élément correcteur de l'acuité visuelle, pendant une durée plus longue.

9. Dispositif selon l'une des revendications 1 à 6, **caractérisé en ce que** le corps de base est optiquement transparent, et
ses surfaces, situées dans un chemin de rayon d'observation, sont configurées de manière que, conjointement avec le pouvoir convergeant ou puissant de l'oeil, il produit une illustration optiquement neutre.

10. Dispositif selon l'une des revendications 1 à 9, **caractérisé par** :
un élément rapporté (39 ; 47) susceptible d'être rapporté, avec une surface d'application (41 ; 51) pouvant être appliquée sur la cornée, pour écranter, de façon stérile et remplaçable, la surface d'application (5 ; 49), non stérile, vis-à-vis de la cornée, sachant que
du matériau (41 ; 53), venant se placer par l'intermédiaire de l'unité sensible à la pression (9), de l'élément rapporté (39 ; 47), est d'une minceur telle que, certes, on obtient une stabilité contre le déchirement mais que, cependant, en cas d'application, la pression est transmissible de la cornée de l'oeil, sans perte, à l'unité sensible à la pression (9).

11. Dispositif selon l'une des revendications 1 à 10, **caractérisé par**
une unité tampon (71) susceptible d'être appliquée, de préférence remplaçable, disposée sur le bord de la surface d'application (5), et qui
et capte l'excès de liquide lacrymal et le refoule hors de la zone de la surface d'application, de manière que la mesure ne soit aucunement influencée.

12. Dispositif selon l'une des revendications 1 à 11, **caractérisé par**
un dispositif (62), présentant une rigidité prédéterminée, reliant l'unité (3), présentant la surface d'application (5), à un site de fixation (63), où
la rigidité doit être choisie de manière que l'unité (3), lorsque la surface d'application (5) est posée sur l'oeil, accompagne au moins de petits mouvements de l'oeil.
